# EUROPEAN PATENT SPECIFICATION

(11) **EP 4 091 559 B1**
(45) Date of publication and mention of the grant of the patent: **22.01.2025**
(21) Application number: 22172981.7
(22) Date of filing: 12.05.2022
(51) Int. Cl.: A61B 17/3207, A61B 17/3205, A61B 17/22, A61B 90/00, A61B 17/00, A61B 17/32

(54) **TISSUE-REMOVING CATHETER WITH GUIDEWIRE ISOLATION LINER**
GEWEBEENTNAHMEKATHETER MIT FÜHRUNGSDRAHTISOLATIONSLINER
CATHÉTER D'ABLATION DE TISSU DOTÉ D'UN REVÊTEMENT D'ISOLATION DE FIL-GUIDE

(30) Priority: 18.05.2021 US 202163190102 P; 02.05.2022 US 202217661669
(43) Date of publication of application: 23.11.2022
(73) Proprietor: Medtronic Vascular, Inc., Santa Rosa, CA 95403 (US)
(72) Inventor: JAMOUS, Aram, Santa Rosa (US); KELLY, Tomas K., Santa Rosa (US); DONEGAN, Michael James, Santa Rosa (US); RYAN, Alan, Santa Rosa (US); JENNINGS, Eoghan, Santa Rosa (US); WALSH, Eoin J., Santa Rosa (US); MURRAY, Aran, Santa Rosa (US)
(74) Representative: Zimmermann & Partner Patentanwälte mbB

(56) References cited:
- US-A1- 2020 078 038
- US-A1- 2020 261 112

## Description

### CROSS-REFERENCE TO RELATED APPLICATION

This application claims the benefit of U.S. Provisional Patent Application Serial No. 63/190,102, filed on May 18, 2021.

### FIELD

The present disclosure generally relates to a tissue-removing catheter, and more particular, to an isolation liner for a tissue-removing catheter.

### BACKGROUND

Tissue-removing catheters are used to remove unwanted tissue in body lumens. As an example, atherectomy catheters are used to remove material from a blood vessel to open the blood vessel and improve blood flow through the vessel. This process can be used to prepare lesions within a patient's coronary artery to facilitate percutaneous coronary angioplasty (PTCA) or stent delivery in patients with severely calcified coronary artery lesions. Atherectomy catheters typically employ a rotating element which is used to abrade or otherwise break up the unwanted tissue.

US 2020/0078038 A1 describes a tissue-removing catheter with guidewire detection sensor. US 2020/0261112 A1 describes a tissue-removing catheter including a turbine.

### SUMMARY OF DISCLOSURE

The invention is defined in claim 1. Further embodiments are defined in the dependent claims. An aspect of the present disclosure provides a tissue-removing catheter for removing tissue in a body lumen. The tissue-removing catheter includes an elongate body, a tissue-removing element, and an inner liner. The elongate body has an axis and proximal and distal end portions spaced apart from one another along the axis. The elongate body is sized and shaped to be received in the body lumen. The tissue-removing element is mounted on the distal end portion of the elongate body and is configured to remove tissue as the tissue-removing element is rotated by the elongate body within the body lumen. The inner liner is received within the elongate body and defines a guidewire lumen. The inner liner isolates an interior of the guidewire lumen from the elongate body and tissue-removing element such that a torsional force is not transferred from the elongate body and tissue-removing element to the interior of the guidewire lumen when the elongate body and tissue-removing element are rotated during operation of the tissue-removing catheter. The inner liner includes a distal end margin configured to extend distally of the tissue-removing element. The distal end margin may have a construction different from a construction of a second portion of the inner liner, which the second portion is positioned proximal of the distal end margin. The distal end margin may be more flexible than the second portion of the inner liner.

### BRIEF DESCRIPTION OF THE DRAWINGS

FIG. 1 is a schematic illustration of a catheter of the present disclosure;
FIG. 2 is an enlarged elevation of a distal end portion of the catheter;
FIG. 3 is a cross section taken through line 3-3 in FIG. 2;
FIG. 4 is a top perspective of a handle of the catheter;
FIG. 5 is a top perspective of the handle with a top housing section removed;
FIG. 6 is a perspective of gears of a gear assembly in the handle;
FIG. 7 is a fragmentary elevation of an isolation liner of the catheter with portions broken away to show internal details;
FIG. 8 is an enlarged fragmentary longitudinal cross section of the distal end portion of the catheter in FIG. 2;
FIG. 9 is an enlarged longitudinal cross section of a tissue-removing element of the catheter;
FIG. 10 is a perspective of a bushing of the catheter;
FIG. 11 is a perspective of a first bearing of the catheter;
FIG. 12 is a perspective of a second bearing of the catheter; and
FIG. 13 is an illustration of the catheter in a body lumen.

Corresponding reference characters indicate corresponding parts throughout the drawings.

### DETAILED DESCRIPTION

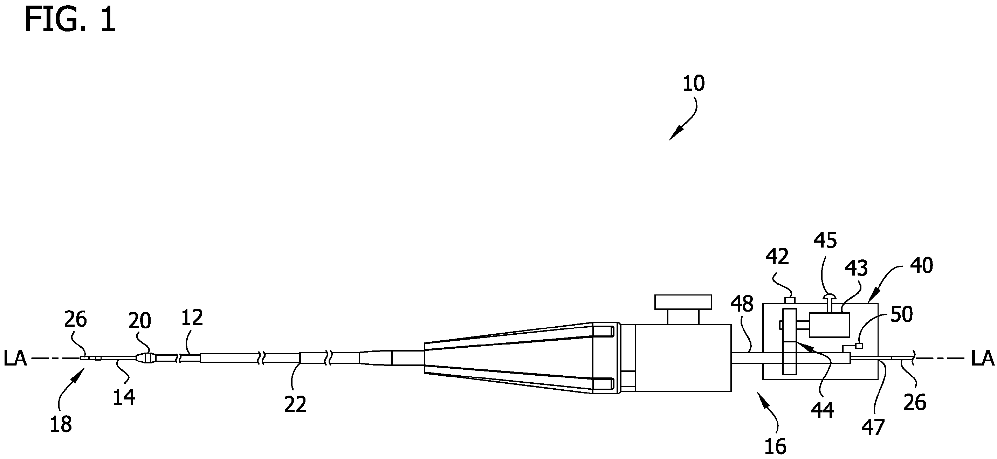

Referring to the drawings, and in particular FIG. 1, a rotational tissue-removing catheter for removing tissue in a body lumen is generally indicated at reference number 10. The illustrated catheter 10 is a rotational atherectomy device suitable for removing (e.g., abrading, cutting, excising, ablating, etc.) occlusive tissue (e.g., embolic tissue, plaque tissue, atheroma, thrombolytic tissue, stenotic tissue, hyperplastic tissue, neoplastic tissue, etc.) from a vessel wall (e.g., coronary arterial wall, etc.). The catheter 10 may be used to facilitate percutaneous coronary angioplasty (PTCA) or the subsequent delivery of a stent. Features of the disclosed embodiments may also be suitable for treating chronic total occlusion (CTO) of blood vessels, and stenoses of other body lumens and other hyperplastic and neoplastic conditions in other body lumens, such as the ureter, the biliary duct, respiratory passages, the pancreatic duct, the lymphatic duct, and the like. Neoplastic cell growth will often occur as a result of a tumor surrounding and intruding into a body lumen. Removal of such material can thus be beneficial to maintain patency of the body lumen.

The catheter 10 is sized for being received in a blood vessel of a subject. Thus, the catheter 10 may have a maximum size of 3, 4, 5, 6, 7, 8, 9, 10, or 12 French (1, 1.3, 1.7, 2, 2.3, 2.7, 3, 3.3, or 4 mm) and may have a working length of 20, 30, 40, 60, 80, 100, 120, 150, 180 or 210 cm depending on the body lumen. While the remaining discussion is directed toward a catheter for removing tissue in blood vessels, it will be appreciated that the teachings of the present disclosure also apply to other types of tissue-removing catheters, including, but not limited to, catheters for penetrating and/or removing tissue from a variety of occlusive, stenotic, or hyperplastic material in a variety of body lumens.

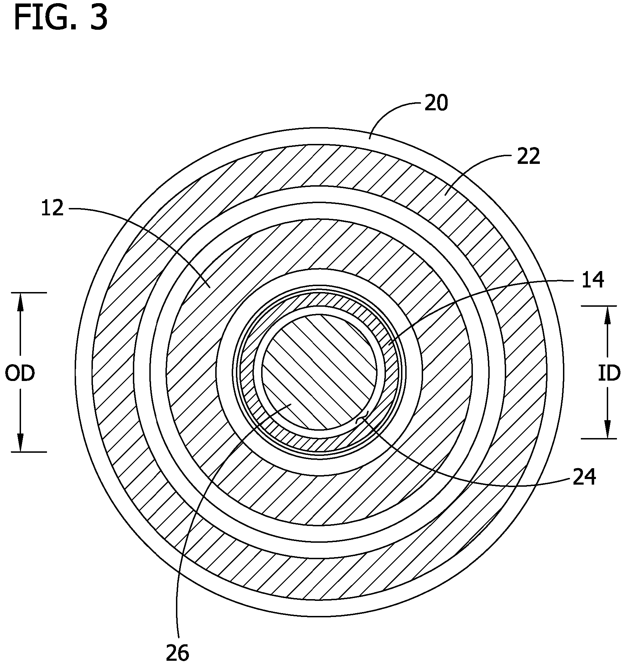

Referring to FIGS. 1-3, the catheter 10 comprises an elongate drive coil 12 (broadly, an elongate body) disposed around an elongate inner liner 14. The drive coil 12 and inner liner 14 extend along a longitudinal axis LA of the catheter from a proximal end portion 16 to a distal end portion 18 of the catheter. A tissue-removing element 20 is disposed on a distal end of the drive coil 12 and is configured for rotation to remove tissue from a body lumen as will be explained in greater detail below. An isolation sheath 22 is disposed around the drive coil 12. The drive coil 12 and the inner liner 14 are both configured to translate relative to the isolation sheath 22. The catheter 10 is sized and shaped for insertion into a body lumen of a subject. The isolation sheath 22 isolates the body lumen from at least a portion of the drive coil 12 and inner liner 14. The inner liner 14 defines a guidewire lumen 24 (FIG. 3) for slidably receiving a guidewire 26 therein so that the catheter 10 can be advanced through the body lumen by traveling along the guidewire. The guidewire can be a standard 0.014-inch (0.36 mm) outer diameter, 300 cm length guidewire. In certain embodiments, the inner liner 14 may have a lubricious inner surface for sliding over the guidewire 26 (e.g., a lubricious surface may be provided by a lubricious polymer layer or a lubricious coating). In the illustrated embodiment, the guidewire lumen 24 extends along an entire working length of the catheter 10. In one embodiment, the overall working length of the catheter 10 may be between about 135 cm (53 inches) and about 142 cm (56 inches). In use, the guidewire 26 may extend by an extension distance, e.g. about 40 mm (1.6 inches), past a distal end of the inner liner 14.

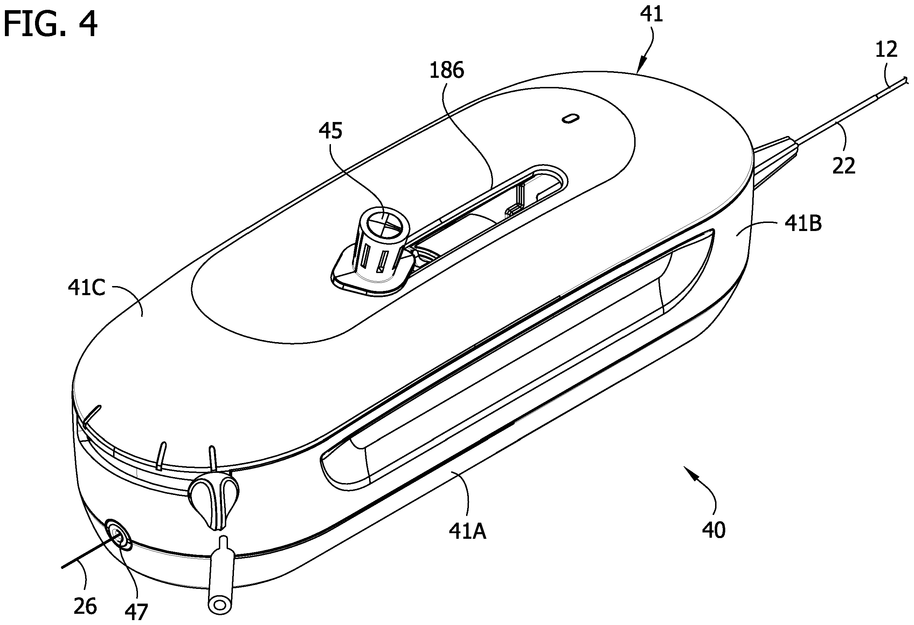

Referring to FIGS. 1 and 4-7, the catheter 10 further comprises a handle 40 secured at a proximal end of the isolation sheath 22. The handle 40 comprises a housing 41 that supports the components of the handle. The housing 41 has a generally elongate egg shape and includes a plurality of housing sections secured together to enclose the internal components of the handle 40. In the illustrated embodiment, the housing 41 includes a bottom housing section 41A, a middle housing section 41B secured to the top of the bottom housing section, and a top housing section 41C secured to the top of the middle housing section. In one embodiment, the bottom housing section 41A is removable from the middle housing section 41B to provide access to the components of the handle 40 in the interior of the housing 41 by a user. It will be understood that the housing 41 can have other shapes and configurations without departing from the scope of the disclosure.

The housing 41 supports an actuator 42 (e.g., a lever, a button, a dial, a switch, or other device) configured for selectively actuating a motor 43 disposed in the handle to drive rotation of the drive coil 12, and the tissue-removing element 20 mounted at the distal end of the drive coil. The motor 43 is configured to rotate the drive coil 12 and tissue-removing element 20 at speeds of greater than about 80,000 RPM. In one embodiment, the motor 43 rotates the drive coil 12 and tissue-removing element 20 between about 10,000 and about 110,000 RPM.

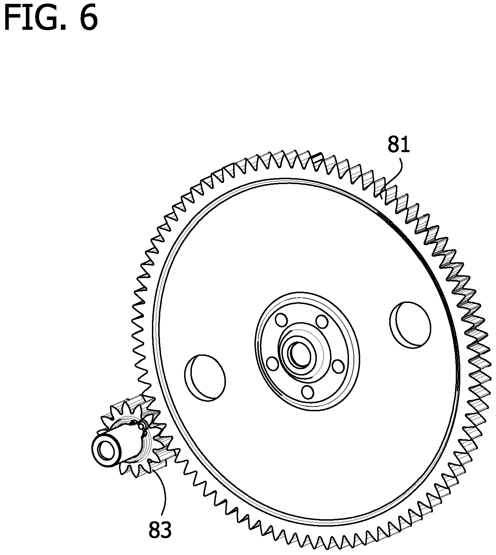

The motor 43 is coupled to the drive coil 12 by a gear assembly 44 and a drive assembly 48 supported within the housing 41. The gear assembly 44 comprises a gearbox housing 55 that mounts and at least partially encloses a pair of gears for transferring the rotation of a shaft of the motor 43 to the drive coil 12. The gearbox housing 55 also attaches to a carriage or advancer frame 73 for moving the motor 43 and gear assembly 44 within the housing 41. Further, attaching the gearbox housing 55 to the distal end of the advancer frame 73 secures the motor 43 in the advancer frame so that the motor moves along with the advancer frame. A driver gear 81 is attached to the motor 43 such that the driver gear rotates with the motor shaft when the motor 43 is activated (FIG. 6). A driven gear 83 is in mesh with the driver gear 81 so that rotation of the driver gear causes the driven gear to rotate in the opposite direction. The drive assembly 48 attaches the driven gear 83 to the drive coil 12 so that the rotation of the driven gear causes the drive coil to rotate. A controller 50 may be provided in the handle 40. The controller 50 may be programmed to control operation of the catheter.

It is understood that other suitable actuators, including but not limited to touchscreen actuators, wireless control actuators, automated actuators directed by a controller, etc., may be suitable to selectively actuate the motor in other embodiments. In some embodiments, a power supply may come from a battery (not shown) contained within the handle 40. The battery can provide the current source for the guidewire detection circuit. In other embodiments, the power supply may come from an external source.

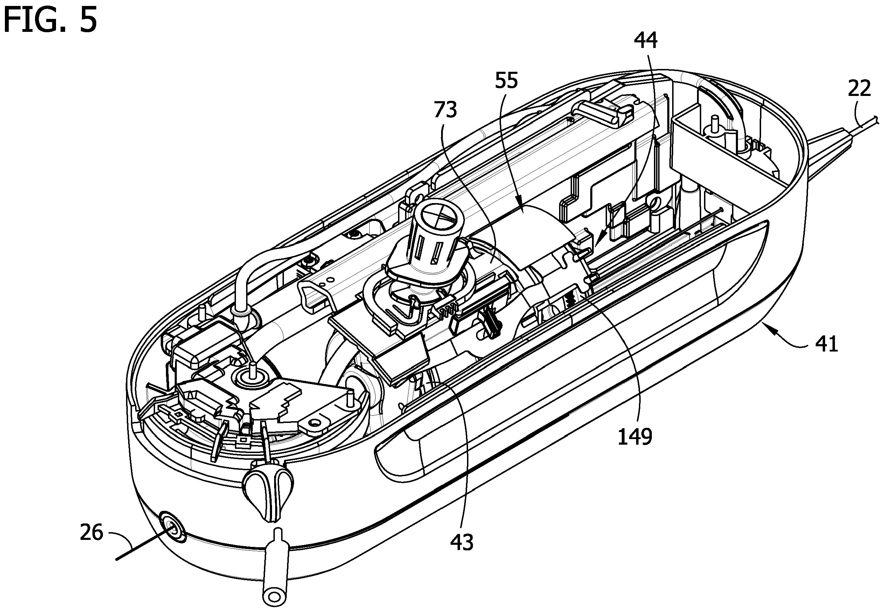

Referring to FIGS. 1, 4, and 5, a slide or advancer 45 is positioned on the handle 40 and is operatively coupled to the inner liner 14 for movement of the inner liner relative to the handle to advance and retract the inner liner, drive coil 12, and tissue-removing element 20. The housing 41 of the handle 40 may define a slot 186 which limits the movement of the slide 45 relative to the handle. Thus, the length of the slot 186 determines the amount of relative movement between the inner liner 14 and the handle 40. In one embodiment, the slot has a length of about 70 mm (2.8 inches). The slide 45 is operatively attached to the advancer frame 73 so that movement of the slide causes movement of the advancer frame. The advancer frame 73 comprises an arch shaped body configured to slidingly receive the cylindrically shaped motor 43. Bearings 149 (FIG. 5) are mounted on the frame 73. The bearings 149 engage the housing 41 so that the bearings can slide along the housing to facilitate movement of the frame 73 in the housing.

Referring to FIGS. 1 and 3, the isolation sheath 22 comprises a tubular sleeve configured to isolate and protect a subject's arterial tissue within a body lumen from the rotating drive coil 12. The isolation sheath 22 is fixed to the handle 40 at a proximal end of the sheath and does not rotate. The isolation sheath 22 provides a partial enclosure for the drive coil 12 and inner liner 14 to move within the sheath. The inner diameter of the isolation sheath 22 is sized to provide clearance for the drive coil 12. The space between the isolation sheath 22 and the drive coil 12 allows for the drive coil to rotate within the sheath and provides an area for saline perfusion between the sheath and drive coil. The outer diameter of the isolation sheath 22 is sized to provide clearance with an inner diameter of a guide catheter (not shown) for delivering the catheter 10 to the desired location in the body lumen. In one embodiment, the isolation sheath 22 has an inner diameter of about 0.050 inches (1.27 mm), an outer diameter of about 0.055 inches (1.4 mm), and a length of about 1500 mm (59 inches). The isolation sheath 22 can have other dimensions without departing from the scope of the disclosure. In one embodiment, the isolation sheath 22 is made from Polytetrafluorethylene (PTFE). Alternatively, the isolation sheath 22 may comprise a multi-layer construction. For example, the isolation sheath 22 may comprise an inner layer of perfluoroalkox (PFA), a middle braided wire layer, and an outer layer of Pebax.

Referring to FIGS. 1-3, the drive coil 12 may comprise a tubular stainless steel coil configured to transfer rotation and torque from the motor 43 to the tissue-removing element 20. Configuring the drive coil 12 as a coiled structure allows for the rotation and torque of the drive coil 12 to be applied to the tissue-removing element 20 when the catheter 10 is traversed across a curved path. The coil configuration of the drive coil 12 is also configured to expand its inner diameter when the coil is rotated so that the drive coil remains spaced from the inner liner 14 during operation of the catheter 10. In one embodiment, the drive coil 12 has an inner diameter of about 0.023 inches (0.6 mm) and an outer diameter of about 0.035 inches (0.9 mm). The drive coil 12 may have a single layer construction. For example, the drive coil may comprise a 7 filar (i.e., wire) coil with a lay angle of about 30 degrees. Alternatively, the drive coil 12 could be configured from multiple layers without departing from the scope of the disclosure. For example, the drive coil 12 may comprise a base coil layer and a jacket (e.g., Tecothane^{™}) disposed over the base layer. In one embodiment, the drive coil comprises a 15 filar coil with a lay angle of about 45 degrees. The Tecothane^{™} jacket may be disposed over the coil. Alternatively, the drive coil 12 may comprise a dual coil layer configuration which also includes an additional jacket layer over the two coil layers. For example, the drive coil may comprise an inner coil layer comprising a 15 filar coil with a lay angle of about 45 degrees, and an outer coil layer comprising a 19 filar coil with a lay angle of about 10 degrees. Drive coils having other configurations are also envisioned.

Referring to FIGS. 1-3 and 7, the inner liner 14 comprises a multiple layer tubular body configured to isolate the guidewire 26 from the drive coil 12 and tissue-removing element 20. The inner liner 14 is extendable through the handle 40 from a position within the handle to a position distal of the handle. In one embodiment, the inner liner 14 is coupled to the components within the handle 40 but is not fixedly attached to the housing 41 to allow translation of the inner liner relative to the housing. In another embodiment, the inner liner 14 is fixedly coupled to components within the handle 40 to prevent translation of the inner liner relative to the housing 41. The inner liner 14 has an inner diameter that is sized to pass the guidewire 26. The inner liner 14 protects the guidewire from being damaged by the rotation of the drive coil 12 by isolating the guidewire from the rotatable drive coil. The inner liner 14 may also extend past the tissue-removing element 20 to protect the guidewire 26 from the rotating tissue-removing element. Thus, the inner liner 14 can be configured to prevent any contact between the guidewire 26 and the rotating components of the catheter 10. Therefore, any metal-to-metal engagement is eliminated by the inner liner 14. This isolation of the drive coil 12 and tissue-removing element 20 from the guidewire 26 also ensures that the rotation of the drive coil and tissue-removing element is not transferred or transmitted to the guidewire. As a result, a standard guidewire 26 can be used with the catheter 10 because the guidewire does not have to be configured to withstand the torsional effects of the rotating components. Additionally, by extending the inner liner 14 through the tissue-removing element 20 and past the distal end of the tissue-removing element, the inner liner stabilizes the tissue-removing element by providing a centering member or axis for rotation of the tissue-removing element.

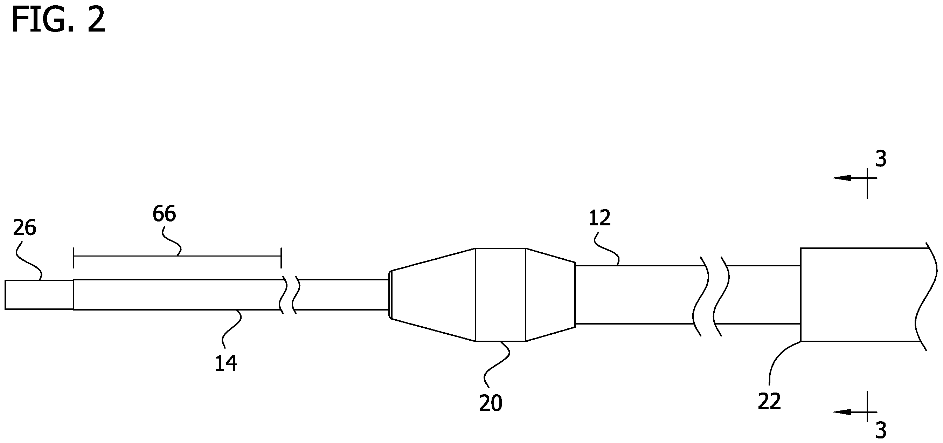

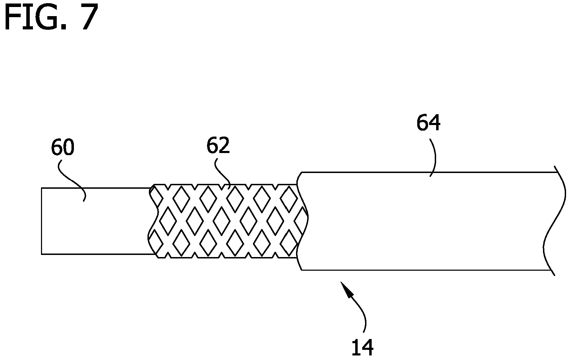

In the illustrated embodiment, the inner liner 14 comprises an inner PTFE layer 60 an intermediate braided layer 62 comprised of stainless steel, and an outer layer 64 of polyimide (FIG. 7). The PTFE inner layer 60 provides the inner liner 14 with a lubricous interior which aids in the passing of the guidewire 26 through the inner liner. The braided stainless steel intermediate layer 62 provides rigidity and strength to the inner liner 14 so that the liner can withstand the torsional forces exerted on the inner liner by the drive coil 12. In one embodiment, the intermediate layer 62 is formed from 304 stainless steel. The outer polyimide layer 64 provides wear resistance as well as having a lubricous quality which reduces friction between the inner liner 14 and the drive coil 12. Additionally, a lubricious film, such as silicone, can be added to the inner liner 14 to reduce friction between the inner liner and the drive coil 12. In one embodiment, the inner layer 60, intermediate layer 62, and outer layer 64 extend along an entire length of the inner liner 14. In one embodiment, at least a portion 66 (FIG. 2) of the inner liner 14 that extends distally of the tissue-removing element 20 is free of at least one of the inner layer 60, intermediate layer 62, or outer layer 64, or a material of at least one of the inner layer, intermediate layer, or outer layer. For example, the portion 66 of the inner liner 14 may be free of the intermediate layer 62 of stainless steel, or generally free of a braided or stainless steel layer/material. In this embodiment, removing the stainless steel layer from the distal end margin of the inner liner 14 increases the flexibility of the inner liner at the distal end margin which facilitates traversing the inner liner through the body lumen of the subject. The portion 66 (e.g., the more flexible distal end margin) also facilitates tracking the catheter 10 on the guidewire and centering the liner 14 and tissue-removing element 20 within a lesion in the body lumen for abrading the lesion. Having the more flexible distal end margin 66 of the inner liner 14 also eliminates the need to attach a separate structure, such as an atraumatic tip, to a distal end of the inner liner. In the illustrated embodiment, the portion 66 extends from a distal end of the inner liner 14 and along only a part of the length of the inner liner that extends distally of the tissue-removing element 20. For example, the portion 66 may extend along less than 3 mm of the inner liner 14. However, the portion 66 may extend along other lengths and be disposed along other sections of the inner liner 14. For example, the portion 66 may extend from the distal end of the inner liner 14 to a location proximal to a distal end of the tissue-removing element 20. In one embodiment, the portion 66 may be spaced from a distal end of the inner liner 14. In one embodiment, the portion 66 of the inner liner 14 may comprise an entire portion of the inner liner extending distally of the tissue-removing element 20.

Referring to FIG. 3, in one embodiment, the inner liner 14 has an inner diameter ID of about 0.016 inches (0.4 mm), an outer diameter OD of about 0.019 inches (0.5 mm), and a length of about 59 inches (1500 mm). The inner diameter ID of the inner liner 14 provides clearance for the standard 0.014-inch (0.36 mm) guidewire 26. The outer diameter OD of the inner liner 14 provides clearance for the drive coil 12 and tissue-removing element 20. The presence of a space between the inner liner 14 and the drive coil 12 reduces friction between the two components as well as allows for saline perfusion between the components.

Referring to FIGS. 1, 2, and 8, the tissue-removing element 20 extends along the longitudinal axis LA from a proximal end adjacent the distal end portion of the drive coil 12 to an opposite distal end. The tissue-removing element 20 is operatively connected to the motor 43 for being rotated by the motor. When the catheter 10 is inserted into the body lumen and the motor 43 is rotating the tissue-removing element 20, the tissue-removing element is configured to remove occlusive tissue in the body lumen to separate the tissue from the wall of the body lumen. Any suitable tissue-removing element for removing tissue in the body lumen as it is rotated may be used in one or more embodiments. In the illustrated embodiment, the tissue-removing element 20 comprises an abrasive burr configured to abrade tissue in the body lumen when the motor 43 rotates the abrasive burr. The abrasive burr 20 has an abrasive outer surface formed, for example, by a diamond grit coating, surface etching, or the like. In other embodiments, the tissue-removing element 20 can comprise one or more cutting elements having smooth or serrated cutting edges, a macerator, a thrombectomy wire, etc.

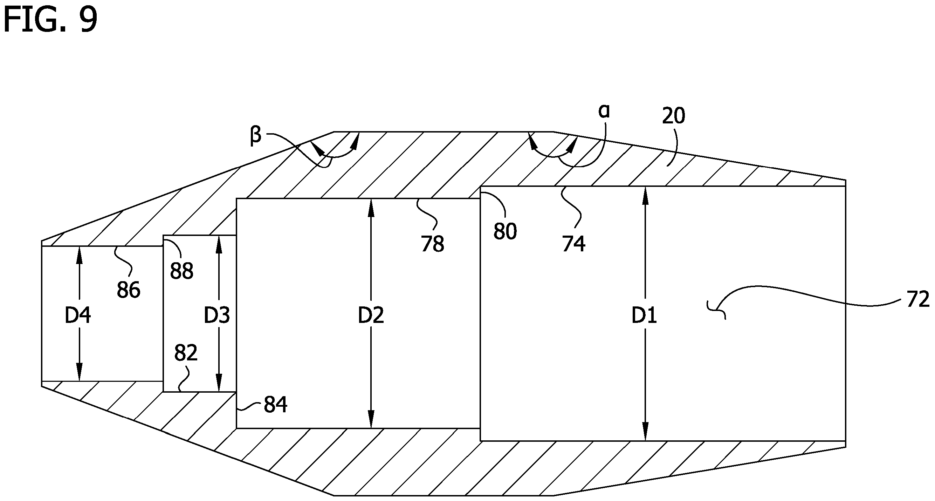

Referring to FIG. 9, a cavity 72 extends longitudinally through the tissue-removing element 20 such that the tissue-removing element defines openings at its proximal and distal ends. The cavity 72 includes a first diameter portion 74 extending distally from the proximal end of the tissue-removing element 20 and a second diameter portion 78 extending distally from the first diameter portion forming a first shoulder 80 disposed between the first and second diameter portions. A third diameter portion 82 extends distally from the second diameter portion 78 and forms a second shoulder 84 between the second and third diameter portions. A fourth diameter portion 86 extends distally from the third diameter portion to the distal end of the tissue-removing element and forms a third shoulder 88 between the third and fourth diameter portions. The diameters of the first, second, third, and fourth diameter portions 74, 78, 82, 86 are constant along their lengths. In the illustrated embodiment, a diameter D1 of the first diameter portion 74 is larger than a diameter D2 of the second diameter portion 78, the diameter D2 is larger than a diameter D3 of the third diameter portion 82, and the diameter D3 is larger than a diameter D4 of the fourth diameter portion 86. In one embodiment, the diameter D1 of the first diameter portion 74 is about 0.037 inches (0.95 mm), the diameter D2 of the second diameter portion 78 is about 0.035 inches (0.9 mm), the diameter D3 of the third diameter portion 82 is about 0.033 inches (0.85 mm), and the diameter D4 of the fourth diameter portion 86 is about 0.031 inches (0.8 mm). Other cross-sectional dimensions are also envisioned without departing from the scope of the disclosure.

The inner liner 14 extends through the drive coil 12 and past the distal end of the tissue-removing element 20. The fourth diameter portion 86 of the cavity 72 is sized to pass the inner liner 14 with a small clearance. The inner diameter D4 provides clearance between the tissue-removing element 20 and the inner liner 14 to reduce friction between the components. Accordingly, the tissue-removing element 20 is shaped and arranged to extend around at least a portion of the drive coil 12 and inner liner 14 and thus provides a relatively compact assembly for abrading tissue at the distal end portion of the catheter 10.

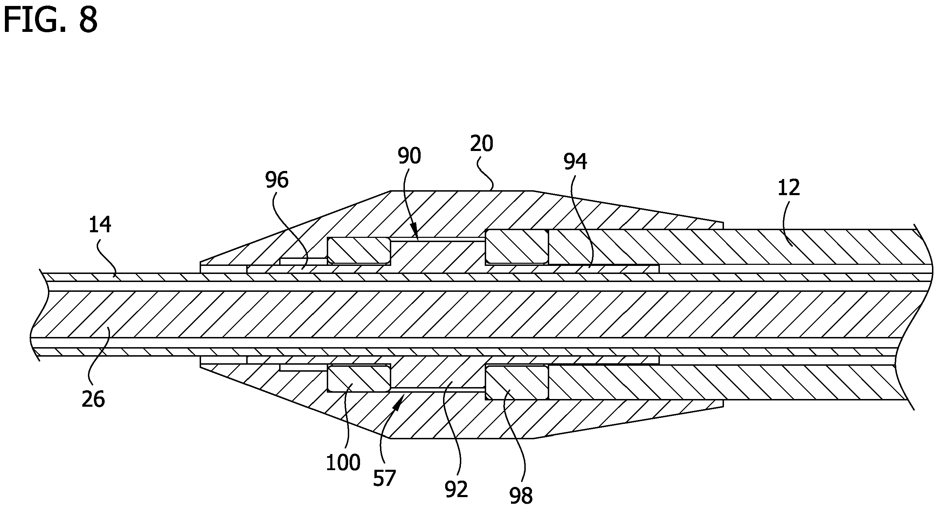

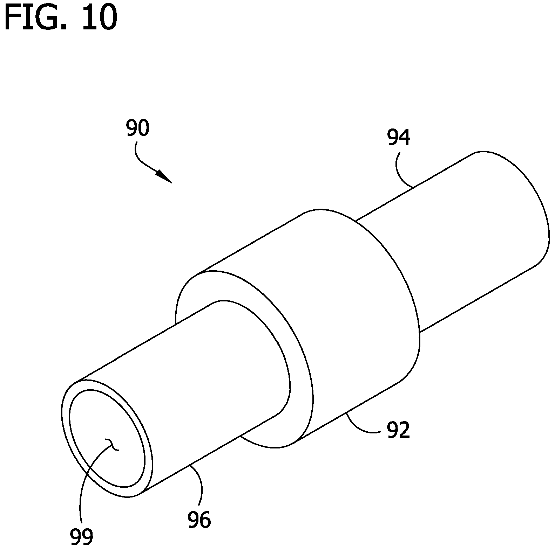

Referring to FIGS. 8-10, a bushing 90 is received in the cavity 72 of the tissue-removing element 20 and around the inner liner 14. The bushing 90 comprises a center ring portion 92, a proximal ring portion 94, extending proximally from the center ring portion, and a distal ring portion 96, extending distally from the center ring portion. The ring portions of the bushing 90 define a channel 99 extending through the bushing that receives a portion of the inner liner 14. In the illustrated embodiment, the center ring portion 92 has a larger outer diameter than the proximal and distal ring portions 94, 96. The center ring portion 92 is disposed in the second diameter portion 78 of the cavity 72, the proximal ring portion 94 is disposed in the first diameter portion 74, and the distal ring portion 96 is disposed in the second and third diameter portions 78, 82. In one embodiment, the bushing 90 is made from polyetheretherketone (PEEK) and polytetrafluoroethylene (PTFE). However, the bushing 90 can be formed from other material without departing from the scope of the disclosure.

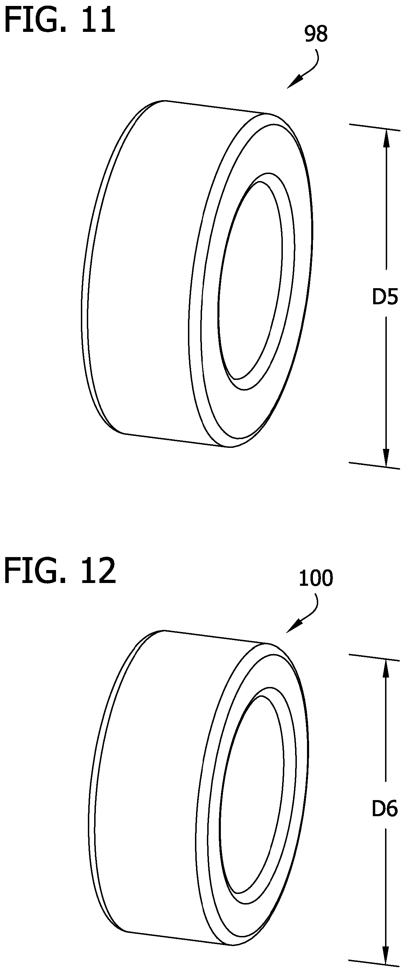

Referring to FIGS. 8, 11, and 12, a first bearing 98 is disposed around the proximal ring portion 94 of the bushing 90, and a second bearing 100 is disposed around the distal ring portion 96 of the bushing. The first bearing 98 has an outer diameter D5 that is greater than an outer diameter D6 of the second bearing 100. In one embodiment, the first and second bearings 98, 100 are made from Zirconia. The first bearing 98 is disposed in registration with the first diameter portion 74 of the cavity 72 in the tissue-removing element 20 and seats between a distal end of the drive coil 12 at a proximal end of the first bearing, and the center ring portion 92 of the bushing 90 and first shoulder 80 at a distal end of the first bearing. The second bearing 100 is disposed in registration with the second diameter portion 78 of the cavity 72 and is seated between the second shoulder 84 at a distal end of the second bearing, and the center ring portion 92 of the bushing 90 at a proximal end of the second bearing. As such the bushing 90 and first and second bearings 98, 100 are held within the cavity 72 of the tissue-removing element 20. Broadly, the bushing 90 and first and second bearings 98, 100 may be considered a coupling assembly 57 for coupling the inner liner 14 to the tissue-removing element 20.

Referring to FIG. 8, an interior surface of the bushing 90 is fixedly attached to the inner liner 14 such that the inner liner is coupled to the tissue-removing element 20 through the bushing. In one embodiment an adhesive such as an epoxy glue bonds the bushing 90 to the inner liner 14. As such, the bushing 90 does not rotate around the inner liner 14. The drive coil 12 is directly and fixedly attached to the tissue-removing element 20. The tissue-removing element 20 can be fixedly attached to the distal end of the drive coil 12 by any suitable means. In one embodiment, adhesive bonds the drive coil 12 to the tissue-removing element 20. The drive coil 12 is received in the first diameter portion 74 of the cavity 72 and a distal end of the drive coil abuts the first bearing 98. However, the inner liner 14 is not directly attached to the tissue-removing element 20, and the drive coil 12 is not directly attached to the bushing 90, first and second bearings 98, 100, or inner liner. Thus, rotation of the drive coil 12 and tissue-removing element 20 is not transmitted to the inner liner 14 to also rotate the inner liner. Rather the tissue-removing element 20 rotates around the bushing 90 and first and second bearings 98, 100. And because the inner liner is fixedly attached to the bushing 90, which is retained within the cavity 72 of the tissue-removing element 20 by the drive coil 12, the inner liner 14 is coupled to the drive coil and tissue-removing element through the bushing and bearing arrangement. It will be understood that the inner liner 14 may be coupled to the tissue-removing element 20 by other means. Alternatively, the inner liner 14 may not be coupled to the tissue-removing element 20.

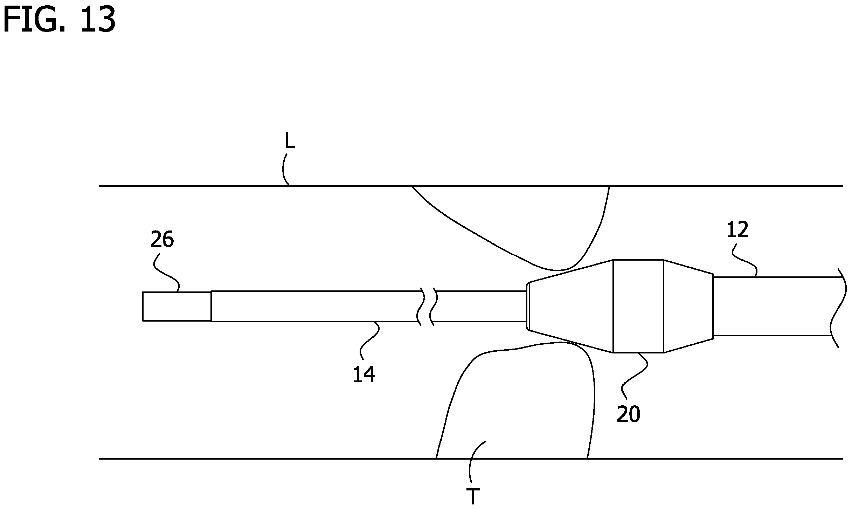

Referring to FIGS. 1, 2, and 13, to remove tissue T in the body lumen L of a subject, a practitioner inserts the guidewire 26 into the body lumen L of the subject, to a location distal of the tissue T that is to be removed. Subsequently, the practitioner inserts the proximal end portion of the guidewire 26 through the guidewire lumen 24 of the inner liner 14 and through the handle 40 so that the guidewire extends through a proximal port 47 in the handle. With the catheter 10 loaded onto the guidewire 26, the practitioner advances the catheter along the guidewire until the tissue-removing element 20 is positioned proximal and adjacent the tissue T. When the tissue-removing element 20 is positioned proximal and adjacent the tissue T, the practitioner actuates the motor 43 using the actuator 42 to rotate the drive coil 12 and the tissue-removing element mounted on the drive coil. The tissue-removing element 20 abrades (or otherwise removes) the tissue T in the body lumen L as it rotates. While the tissue-removing element 20 is rotating, the practitioner may selectively move the drive coil 12 and inner liner 14 distally along the guidewire 26 to abrade the tissue T and, for example, increase the size of the passage through the body lumen L. The practitioner may also move the drive coil 12 and inner liner 14 proximally along the guidewire 26, and may repetitively move the components in distal and proximal directions to obtain a back-and-forth motion of the tissue-removing element 20 across the tissue T by sliding the advancer 45 back and forth within the slot 186 in the handle 40. During the abrading process, the inner liner 14 isolates the guidewire 26 from the rotating drive coil 12 and tissue-removing element 20 to protect the guidewire from being damaged by the rotating components. As such, the inner liner 14 is configured to withstand the torsional and frictional effects of the rotating drive coil 12 and tissue-removing element 20 without transferring those effects to the guidewire 26. When the practitioner is finished using the catheter 10, the catheter can be withdrawn from the body lumen L and unloaded from the guidewire 26 by sliding the catheter proximally along the guidewire. The guidewire 26 used for the abrading process may remain in the body lumen L for use in a subsequent procedure.

When introducing elements of the present invention or the one or more embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above apparatuses and systems without departing from the scope of the invention, it is intended that all matter contained in the above description and shown in the accompanying drawings shall be interpreted as illustrative and not in a limiting sense. The features of various embodiments can be combined to form further embodiments of the invention that may not be explicitly described or illustrated. Each embodiment and each aspect so defined may be combined with any other embodiment or with any other aspect unless clearly indicated to the contrary.

## Claims

1. A tissue-removing catheter (10) for removing tissue in a body lumen, the tissue-removing catheter comprising:
an elongate body (12) having an axis (LA) and proximal and distal end portions (16, 18) spaced apart from one another along the axis, the elongate body being sized and shaped to be received in the body lumen;
a tissue-removing element (20) mounted on the distal end portion of the elongate body, the tissue-removing element being configured to remove tissue as the tissue-removing element is rotated by the elongate body within the body lumen; and
an inner liner (14) received within the elongate body and defining a guidewire lumen (24), the inner liner isolating an interior of the guidewire lumen from the elongate body and tissue-removing element such that torsional force is not transferred from the elongate body and tissue-removing element to the interior of the guidewire lumen when the elongate body and tissue-removing element are rotated during operation of the tissue-removing catheter, the inner liner including a distal end margin (66) configured to extend distally of the tissue-removing element, the distal end margin having a construction different from a construction of a second portion of the inner liner which is positioned proximal of the distal end margin, and wherein the distal end margin of the inner liner is more flexible than the remaining portion of the inner liner.

2. A tissue-removing catheter as set forth in claim 1, wherein the inner liner comprises a plurality of layers (60, 62, 64) each extending from a proximal end of the inner liner toward a distal end of the inner liner.

3. A tissue-removing catheter as set forth in any preceding claim, wherein the distal end margin has a fewer number of layers than the second portion of the inner liner.

4. A tissue-removing catheter as set forth in any preceding claim, wherein the guidewire lumen extends through the distal end of the inner liner.

5. A tissue-removing catheter as set forth in any preceding claim, wherein the inner liner includes an inner layer (60), an outer layer (64), and an intermediate layer (62) disposed between the inner and outer layers.

6. A tissue-removing catheter as set forth in claim 5, wherein the distal end margin includes the inner layer and outer layer, the distal end margin being free of the intermediate layer; and/or wherein the inner layer comprises Polytetrafluorethylene (PTFE), the intermediate layer comprises stainless steel, and the outer layer comprises polyimide.

7. A tissue-removing catheter as set forth in any preceding claim, wherein the inner liner includes a metallic component, the distal end margin being free of metal; and optionally wherein the metallic component comprises stainless steel.

8. A tissue-removing catheter as set forth in any preceding claim, wherein a distal end of the inner liner is free of a separate structure attached to the distal end.

9. A tissue-removing catheter as set forth in any preceding claim, wherein the distal end margin extends from a distal end of the inner liner to a location proximal to a distal end of the tissue-removing element.

10. A tissue-removing catheter as set forth in any preceding claim, further comprising a handle (40) mounted on to the proximal end portion of the elongate body and operable to cause rotation of the elongate body; and optionally further comprising a motor (43) in the handle and operatively engaging the elongate body for driving rotation of the elongate body and tissue-removing element mounted on the elongate body.

11. A tissue-removing catheter as set forth in any preceding claim, wherein the tissue-removing element comprises an abrasive burr (20).

12. A tissue-removing catheter as set forth in any preceding claim, wherein the second portion of the inner liner comprises an entirety of the inner liner extending proximally of the distal end margin of the inner liner.

## Patentansprüche

1. Gewebeentfernender Katheter (10) zum Entfernen von Gewebe in einem Körperlumen, der gewebeentfernende Katheter umfassend:
einen länglichen Körper (12), der eine Achse (LA) und proximale und distale Endabschnitte (16, 18) aufweist, die entlang der Achse voneinander beabstandet sind, wobei der längliche Körper bemessen und geformt ist, um in dem Körperlumen aufgenommen zu werden;
ein gewebeentfernendes Element (20), das auf dem distalen Endabschnitt des länglichen Körpers montiert ist, wobei das gewebeentfernende Element konfiguriert ist, um Gewebe zu entfernen, wenn das gewebeentfernende Element durch den länglichen Körper innerhalb des Körperlumens gedreht wird; und
eine Innenauskleidung (14), die innerhalb des länglichen Körpers aufgenommen ist und ein Führungsdrahtlumen (24) definiert, wobei die Innenauskleidung ein Inneres des Führungsdrahtlumens von dem länglichen Körper und dem gewebeentfernenden Element derart isoliert, dass keine Torsionskraft von dem länglichen Körper und dem gewebeentfernenden Element auf das Innere des Führungsdrahtlumens übertragen wird, wenn der längliche Körper und das gewebeentfernende Element während des Betriebs des gewebeentfernenden Katheters gedreht werden, wobei die Innenauskleidung einen distalen Endrand (66) einschließt, der konfiguriert ist, um sich distal des gewebeentfernenden Elements zu erstrecken, wobei der distale Endrand eine andere Konstruktion als eine Konstruktion eines zweiten Abschnitts der Innenauskleidung aufweist, der proximal des distalen Endrands positioniert ist, und wobei der distale Endrand der Innenauskleidung flexibler als der verbleibende Abschnitt der Innenauskleidung ist.

2. Gewebeentfernender Katheter nach Anspruch 1, wobei die Innenauskleidung eine Vielzahl von Schichten (60, 62, 64) umfasst, die sich jeweils von einem proximalen Ende der Innenauskleidung zu einem distalen Ende der Innenauskleidung erstrecken.

3. Gewebeentfernender Katheter nach einem der vorstehenden Ansprüche, wobei der distale Endrand eine geringere Anzahl von Schichten als der zweite Abschnitt der Innenauskleidung aufweist.

4. Gewebeentfernender Katheter nach einem der vorstehenden Ansprüche, wobei sich das Führungsdrahtlumen durch das distale Ende der Innenauskleidung erstreckt.

5. Gewebeentfernender Katheter nach einem der vorstehenden Ansprüche, wobei die Innenauskleidung eine Innenschicht (60), eine Außenschicht (64) und eine Zwischenschicht (62) umfasst, die zwischen der Innen- und der Außenschicht angeordnet ist.

6. Gewebeentfernender Katheter nach Anspruch 5, wobei der distale Endrand die Innenschicht und die Außenschicht einschließt und der distale Endrand frei von der Zwischenschicht ist; und/oder wobei die Innenschicht Polytetrafluorethylen (PTFE) umfasst, die Zwischenschicht Edelstahl umfasst und die Außenschicht Polyimid umfasst.

7. Gewebeentfernender Katheter nach einem der vorstehenden Ansprüche, wobei die Innenauskleidung eine metallische Komponente einschließt und der distale Endrand frei von Metall ist; und optional wobei die metallische Komponente Edelstahl umfasst.

8. Gewebeentfernender Katheter nach einem der vorstehenden Ansprüche, wobei ein distales Ende der Innenauskleidung frei von einer separaten Struktur ist, die an dem distalen Ende angebracht ist.

9. Gewebeentfernender Katheter nach einem der vorstehenden Ansprüche, wobei sich der distale Endrand von einem distalen Ende der Innenauskleidung zu einer Stelle proximal zu einem distalen Ende des gewebeentfernenden Elements erstreckt.

10. Gewebeentfernender Katheter nach einem der vorstehenden Ansprüche, ferner umfassend einen Griff (40), der an dem proximalen Endabschnitt des länglichen Körpers montiert ist und betriebsfähig ist, um eine Drehung des länglichen Körpers zu bewirken; und optional ferner umfassend einen Motor (43) in dem Griff und betriebsmäßiges Ineingriffnehmen des länglichen Körpers zum Antreiben der Drehung des länglichen Körpers und des gewebeentfernenden Elements, das an dem länglichen Körper montiert ist.

11. Gewebeentfernender Katheter nach einem der vorstehenden Ansprüche, wobei das gewebeentfernende Element einen Schleifbohrer (20) umfasst.

12. Gewebeentfernender Katheter nach einem der vorstehenden Ansprüche, wobei der zweite Abschnitt der Innenauskleidung eine Gesamtheit der Innenauskleidung umfasst, die sich proximal des distalen Endrands der Innenauskleidung erstreckt.

## Revendications

1. Cathéter d'ablation de tissu (10) pour pratiquer l'ablation d'un tissu dans une lumière corporelle, le cathéter d'ablation de tissu comprenant :
un corps allongé (12) ayant un axe (LA) et des parties d'extrémité proximale et distale (16, 18) espacées l'une de l'autre le long de l'axe, le corps allongé étant dimensionné et formé pour être reçu dans la lumière corporelle ;
un élément d'ablation de tissu (20) monté sur la partie d'extrémité distale du corps allongé, l'élément d'ablation de tissu étant conçu pour pratiquer l'ablation du tissu lorsque l'élément d'ablation de tissu est tourné par le corps allongé à l'intérieur de la lumière corporelle ; et
un revêtement intérieur (14) reçu à l'intérieur du corps allongé et définissant une lumière de fil-guide (24), le revêtement intérieur isolant un intérieur de la lumière de fil-guide du corps allongé et de l'élément d'ablation de tissu de telle sorte que la force de torsion n'est pas transférée du corps allongé et de l'élément d'ablation de tissu à l'intérieur de la lumière de fil-guide lorsque le corps allongé et l'élément d'ablation de tissu sont tournés pendant l'utilisation du cathéter d'ablation de tissu, le revêtement intérieur comportant un bord d'extrémité distal (66) conçu pour s'étendre de manière distale de l'élément d'ablation de tissu, le bord d'extrémité distale ayant une construction différente d'une construction d'une seconde partie du revêtement intérieur qui est positionné à proximité du bord d'extrémité distale, et dans lequel le bord d'extrémité distale du revêtement intérieur est plus flexible que la partie restante du revêtement intérieur.

2. Cathéter d'ablation de tissu selon la revendication 1, dans lequel le revêtement interne comprend une pluralité de couches (60, 62, 64) s'étendant chacune d'une extrémité proximale du revêtement intérieur vers une extrémité distale du revêtement intérieur.

3. Cathéter d'ablation de tissu selon l'une quelconque revendication précédente, dans lequel le bord d'extrémité distale a un nombre de couches inférieur à celui de la seconde partie du revêtement intérieur.

4. Cathéter d'ablation de tissu selon l'une quelconque revendication précédente, dans lequel la lumière de fil-guide s'étend à travers l'extrémité distale du revêtement intérieur.

5. Cathéter d'ablation de tissu selon l'une quelconque revendication précédente, dans lequel le revêtement intérieur comporte une couche interne (60), une couche externe (64) et une couche intermédiaire (62) disposée entre les couches interne et externe.

6. Cathéter d'ablation de tissu selon la revendication 5, dans lequel le bord d'extrémité distale comporte la couche interne et la couche externe, le bord d'extrémité distale étant libre de la couche intermédiaire ; et/ou dans lequel la couche interne comprend du polytétrafluoréthylène (PTFE), la couche intermédiaire comprend de l'acier inoxydable et la couche externe comprend du polyimide.

7. Cathéter d'ablation de tissu selon l'une quelconque revendication précédente, dans lequel le revêtement intérieur comporte un composant métallique, le bord d'extrémité distale étant exempt de métal ; et éventuellement dans lequel le composant métallique comprend de l'acier inoxydable.

8. Cathéter d'ablation de tissu selon l'une quelconque revendication précédente, dans lequel une extrémité distale du revêtement intérieur est libre d'une structure séparée attachée à l'extrémité distale.

9. Cathéter d'ablation de tissu selon l'une quelconque revendication précédente, dans lequel le bord d'extrémité distale s'étend d'une extrémité distale du revêtement intérieur à un emplacement proximal par rapport à une extrémité distale de l'élément d'ablation de tissu.

10. Cathéter d'ablation de tissu selon l'une quelconque revendication précédente, comprenant en outre une poignée (40) montée sur la partie d'extrémité proximale du corps allongé et utilisable pour provoquer la rotation du corps allongé ; et éventuellement comprenant en outre un moteur (43) dans la poignée et engageant fonctionnellement le corps allongé pour entraîner la rotation du corps allongé et l'élément d'ablation de tissu monté sur le corps allongé.

11. Cathéter d'ablation de tissu selon l'une quelconque revendication précédente, dans lequel l'élément d'ablation de tissu comprend une fraise abrasive (20).

12. Cathéter d'ablation de tissu selon l'une quelconque revendication précédente, dans lequel la seconde partie du revêtement intérieur comprend une totalité du revêtement intérieur s'étendant de manière proximale du bord d'extrémité distale du revêtement intérieur.
